(19) Europäisches Patentamt / European Patent Office / Office européen des brevets

(11) EP 0 938 571 B1

(12) **EUROPEAN PATENT SPECIFICATION**

(45) Date of publication and mention of the grant of the patent:
**07.05.2008 Bulletin 2008/19**

(21) Application number: **97948802.0**

(22) Date of filing: **24.10.1997**

(51) Int Cl.:
***C12N 15/62*** *(2006.01)* ***C07K 19/00*** *(2006.01)*

(86) International application number:
**PCT/EP1997/005897**

(87) International publication number:
**WO 1998/018943 (07.05.1998 Gazette 1998/18)**

(54) **METHOD FOR THE OLIGOMERISATION OF PEPTIDES**

VERFAHREN ZUR OLIGOMERISATION VON PEPTIDEN

TECHNIQUE D'OLIGOMERISATION DE PEPTIDES

(84) Designated Contracting States:
**AT BE CH DE DK ES FI FR GB GR IE IT LI LU MC NL PT SE**

(30) Priority: **28.10.1996 EP 96810719**

(43) Date of publication of application:
**01.09.1999 Bulletin 1999/35**

(73) Proprietors:
- **Novartis AG**
  **4056 Basel (CH)**
  Designated Contracting States:
  **BE CH DE DK ES FI FR GB GR IE IT LI LU MC NL PT SE**
- **University of Lausanne**
  **1066 Epalinges (CH)**
- **Swiss Institute for Allergy and Asthma Research**
  **7270 Davos (CH)**
- **INSTITUT SUISSE DE RECHERCHES EXPERIMENTALES SUR LE CANCER ISREC**
  **1066 Epalinges s/Lausanne (CH)**
- **Novartis Pharma GmbH**
  **1230 Wien (AT)**
  Designated Contracting States:
  **AT**

(72) Inventors:
- **TERSKIKH, Alexey**
  **CH-1018 Lausanne (CH)**
- **CRAMERI, Reto**
  **CH-7270 Davos Platz (CH)**
- **MACH, Jean-Pierre**
  **CH-1010 Lausanne (CH)**
- **LE DOUSSAL, Jean-Marc**
  **F-75008 Paris Cedex (FR)**
- **KAJAVA, Andrey**
  **CH-1007 Lausanne (CH)**

(74) Representative: **de Weerd, Petrus G.W. et al**
**Novartis International AG**
**Corporate Intellectual Property**
**4002 Basel (CH)**

(56) References cited:
**WO-A-95/26985**
**WO-A-95/31540**
**WO-A-96/37621**

- **A.V. KAJAVA: "Modeling of a five-stranded coiled coil structure for the assembly domain of the cartilage oligomeric matrix protein" PROTEINS, vol. 24, no. 2, February 1996, pages 218-226, XP000647810**
- **V.P. EFIMOV ET AL.: "The thrombospondin-like chains of cartilage oligomeric matrix protein are assembled by a five stranded alpha-helical bundle between residued 20 and 83" FEBS LETTERS, vol. 341, 1994, pages 54-58, XP002017642**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Great strides made over the past years in understanding of molecular interactions in the realm of biomolecules such as proteins and nucleic acids benefited from the isolation of artificial polypeptide "ligands" with *de novo* binding activities to "receptors". A powerful means of evolving artificial ligands is offered by the use of large polypeptide libraries, displayed on the surface of filamentous bacteriophage as a fusion with the phage coat proteins. In particular, isolation of new peptide ligands allowed, for example, to map antibodies binding sites, to find an important residues in HLA-DR molecules, to identify proteases substrates and inhibitors. Obviously, isolation of new peptide ligands with high affinity towards their target "receptors" can help to answer important biological questions and is of great interest for pharmaceutical development. However, apart from some exceptions, only low affinity (micromolar range) ligands were isolated from peptide libraries. This can be readily explained by a high degree of conformational freedom and a few number of contact residues within a short peptide molecule.

In order to improve this situation, a new type of high avidity binding molecule is constructed by harnessing the effect of multivalent interaction. Efimov et al. (Federation of European Biochemical Societies Letters, Vol. 341, pp. 54-58, 1994) described the Cartilage Oligomeric Matrix Protein (COMP) and its assembly domain. Argos (Journal of Molecular Biology, Vol. 211, pp. 943-958) further describes the use of linkers to conjugate molecules, said linkers being not susceptible to cleavage by host proteases. The patent application WO9531540 discloses a trimeric protein comprising a neck-region peptide as the oligomerising domain. In the current examples a short peptide ligand is expressed via a semi-rigid hinge region as a fusion with a coiled coil domain from Cartilage Oligomeric Matrix Protein (COMP), resulting in a pentameric multivalent binding molecule. In the case of a Pentabody (Pab-S) as described here, a peptide ligand (S) specific for the mouse B-cell lymphoma ($BCL_1$) surface idiotype, is selected from a phage displayed library. Pab-S molecule specifically bind the $BCL_1$ cells surface idiotype with an apparent avidity of about 1nM, which corresponds to a $2x10^5$ fold increase, compared to the affinity of the synthetic peptide S itself. Equilibrium binding studies strongly suggest that high avidity of Pab-S is a result of multivalent interaction of its "peptide heads" and surface $BCL_1$ immunoglobulin receptor. As demonstrated by gel filtration chromatography, SDS-PAGE analysis and circular dichroism, Pab-S is a stable homopentamer of about 85kDa, with the five chains crosslinked by disulfide bridges. Pab-S can be reversibly denatured, reconstituting upon renaturation its pentameric structure and full binding activity, providing an easy way to bring different peptide specificity within the same heteropentamer, thus bispecific or multispecific pentabodies.

The inventive oligomers have various unique features.

- Target molecule specificity of inventive oligomer can be provided by a short peptide ligand, representing a "minimal" binding domain, where the primary structural information (i.e. the amino acid sequence) is sufficient for recognition. This demonstrates for the first time, that a low affinity peptide ligand (though specific) can be used to create a high avidity binding molecule.
- A hinge region which dictates the geometry and the dynamic features of multivalent interaction can be easily introduced.
- The inventive oligomers can be produced without extensive post translational modifications and, hence, can be produced easily in microorganisms like *E. coli.*
- The peptide ligands can be attached to the C- or N-terminus of the oligomerizing domain, thus providing various binding geometry, with maximal distance between two peptide ligands (for example some attached to the C-terminus and some to the N-terminus in one oligomer).
- If coiled coil $\alpha$-helical domains are used, the resulting oligomers are in most cases well soluble due to the intrinsic structure.
- The COMP domain can be used generally for oligomerization of compounds bound thereto.
- It is a general method for the oligomerization of peptides

### Detailed description of the invention

The present invention concerns an oligomer comprising 2 or more than 2 units, wherein each unit comprises a peptidic domain capable of oligomerizing and a domain capable of binding to an acceptor (ligand), wherein the oligomerizing domain is not an antibody or a functional antibody fragment from the constant region.

A functional antibody fragment is, for example, the $F_c$ domain or the constant region of the light or heavy chain.

In a preferred embodiment of the invention the inventive oligomer comprises more than 2 preferably more than 4 units and most preferably the inventive oligomer consist of 5 units.

In another preferred embodiment the individual units oligomerize spontaneously. According to the present invention, the peptidic domain capable of oligomerizing is the pentamerization domain of the Cartilage Oligomeric Matrix Protein (Slavin & Strober (1978) Nature 272, 624-626) capable of assembling monomeric units. Especially preferred units can be separated from the oligomer and re-oligomerized without loss of activity.

In a further preferred embodiment of the invention each of monomeric units has less that 600 amino acids.
The acceptor, to which the inventive oligomer binds, may be of various origin like, for example, any protein that specifically binds to a partner like a substrate, an inhibitor, an activator, an antibody, a receptor and the like. A preferred acceptor is, for example, an antibody or a receptor. Examples for such preferred antibodies or receptors are $BCL_1$ cells from a B-cell lymphoma surface idiotype or any other receptor expressed on cell surface.
The inventive oligomer may comprise units having the same or different specificities in order to bind to the same or to distinct acceptors; wherein the domain capable of binding to an acceptor can be attached to the C- and/or N-terminus of the oligomerizing domain.
Embraced by the scope of the present invention is also the individual unit capable of oligomerizing as described above, a method for the synthesis of this unit, the expression vector used for this synthesis, as well as the host comprising said expression vector. Preferred hosts are of microbiological origin like *E. coli.*
There are several ways to synthesize the inventive units.

- Via genetic engineering: An expression vector is constructed comprising an expression cassette for the complete inventive unit, that is expressed in a suitable host. Suitable expression cassettes can be prepared by standard techniques in genetic engineering. Beside the information necessary for the synthesis of the desired unit, the expression cassette may additionally contain a signal sequence that forces the secretion of the protein produced. It is also possible to express the inventive unit as a fusion protein with phage coat protein, especially those from filamentous phages and phagemids
- Chemically: Due to the relatively short length of the individual units, they can be synthesized chemically, e.g. via solid phase synthesis.
- Mixed: A part of the inventive unit is expressed by a suitable host and is connected with a chemically synthesized part. For example, the oligomerizing part is synthesized by a microorganism and is elongated by chemical synthesis to add a domain capable of binding to an acceptor.

The peptidic domain capable of oligomerizing and the domain capable of binding to an acceptor may be connected directly or via a spacer (hinge region) to provide, for example, a greater flexibility of the binding domain. For example, a proline-rich sequence of a spacer is supposed to prevent formation of secondary structure elements and as a consequence a fixed 3-D structure. Moreover, a spacer region like that is generally supposed to be quite rigid due to the conformational constrains of the proline residues, bringing a beneficial effect for the cooperativity of the multivalent binding. Accordingly, in another preferred embodiment of the invention, the peptidic domain capable of oligomerizing and the domain capable of binding to an acceptor are connected via a spacer, that comprises preferably a proline-rich region.
Furthermore, the C-terminus of some or all units of the oligomer may be modified by the addition of a further functional domain, like a marker, an enzymatic or a cytotoxic domain, or a domain that adds additional binding properties, for example to metal atoms or other structural compounds, that can be used, e.g., in affinity chromatography.
The individual units may be connected via disulfide bonds, e.g. spontaneously, or via one or more linker molecules. Such linker molecules are molecules bearing two or more reactive groups like -SH, $-N_3$, -COOH, -COBr, -COCl, $-NH_2$, -CHO, -CO-O-CO-, -CO-NH-CO-. Examples are N-5-azido-2-nitrobenzoyloxysuccinimide, p-azidophenazylbromide, p-azidophenyl glyoxal, N-4-(azidophenylthio)phthalimide, bis(sulfosuccinimidyl) suberate, bis-maleimidohexane, bis[2-(succinimidooxycarbonyloxy)ethyl] sulfone, 1,5-difluoro-2,4-dinitrobenzene, 4,4'-diisothiocyano-2,2'-disulfonic acid stilbene, dimethyl adipimidate, dimethyl pimelimidate, dimethyl suberimidate, dithiobis(succinimidylpropionate), disuccinimidyl suberate, disuccinimidyl tatrate, dimethyl 3,3'-dithiobispropionimidate, 4,4'-dithiobisphenylazide, 3,3'-dithiobis (succinimidylpropionate), ethyl-4-azidophenyl-1,4-dithiobutyrimidate, 1-azido-4-fluoro-3-nitrobenzene, N-hydroxysuccinimidyl-4-azidobenzoate, methyl-4-azidobenzoimidate, m-maleimidobenzoyl-N-hydroxysulfo-succinimide ester, N-hydroxysuccinimidyl-4-azidosalicylic acid, p-nitrophenyt-2-diazo-3,3,3-trifluoro propionate, N-succinimidyl(4-azidophenyl)-1,3'-dithiopropionate, sulfosuccinimidyl 2-(m-azido-o-nitrobenzamido)-ethyl-1, 3'-dithiopropionate, N-succinimidyl-6(4'-azido-2'-nitrophenyl-amino)hexanoate, sulfosuccinimidyl 2-(p-azidosalicylamido)ethyl-1,3'-dithiopropionate, N-succinimidyl(4-iodoacetyl)aminobenzoate, succinimidyl 4-(N-maleimidomethyl)-cyclohexane-1-carboxylate, succinimidyl 4-(p-meleimidophenyl)-butyrate, N-succinimidyl 3-(2-pyridyldithio)propionate, bis[2-(sulfosuccinimidooxy-carbonyloxy)ethyl]sulfone, disulfosuccinimidyl tatrate, ethylene glycol-bis(sulfosuccinimidylsuccinate), m-maleimidobenzoyl-N-hydroxysulfosuccinate), sulfosuccinimidyl (4-azidophenyldithio)-propionate, sulfosuccinimidyl 6-(4'azido-2'-nitrophenylamino) hexanoate, sulfosuccinimidyl (4-iodoacetyl)aminobenzoate, sulfosuccinimidyl 4-(N-maleimidomethyl) cyclohexane-1-carboxylate, sulfosuccinimidyl 4-(p-maleimidophenyl)butyrate and 2-iminothiolane.
Preferably, oligomerizing units capable of self-assembling are used. The linker molecules mentioned above may be also used additionally to stabilize the oligomer.
As shown in the example, spontaneous pentamerization of COMP assembly domain does not depend on disulfide bond formation, therefore, a preferred embodiment of the inventive oligomer represents a self-assembling molecule able to oligomerize *in vivo* and/or *in vitro.* Thus oligomerization of short peptides bypass folding problems and overcomes expression difficulties previously experienced during oligomerization of relatively complex proteins such as single chain Fv fragments. Moreover, a self-assembling of an oligomer allows it to undergo a reversible denaturation. Thus, heterooligomers can be readily obtained by mixing the inventive units with different specificity (i.e. with different peptide ligands) under denaturing conditions followed, e.g., by dialysis against any physiological buffer. This intrinsic property of the inventive oligomers opens an easy way to produce a chelating oligomer where two or more peptides directed against different epitopes on the same target molecule would be assembled together into a heteropentameric oligomer, allowing to benefit from the power of chelating effect, as it is recently demonstrated for the single chain Fv fragments. The same procedure can be used to produce heterooligomers with affinity to two or more receptors.

Binding studies of $^{125}$I labeled Pab-S on BCL1 cells, for example, reveal an avidity of about 1 nM for the surface idiotype which is 2 x $10^5$ fold higher compared to $IC_{50}$ of the peptide S itself (200 μM). Pab-S can be reversible denatured (4M, urea 95°C, 15 min), regaining full binding activity after renaturation by dialysis in PBS. Data show that Pab-S has remarkable thermostability, retaining the binding activity even after autoclavation (120°C, 20 min).

Furthermore, it is expected that an oligomer harboring a peptide ligand with a nanomolar intrinsic affinity may attain a femtomolar range of avidity, approaching that of streptavidin-biotin interaction

Based on the properties of the inventive compounds, there are several applications for the inventive oligomers, that are all part of the invention.

- Based on the equilibrium binding results of the example Pab-S it is evident that inventive oligomers are excellent molecules for eukaryotic cell, bacteria or viruses targeting; and especially for cell targeting. As it is shown for Pab-S, the avidity for the cell-surface immobilized receptor is much greater than the avidity for the soluble form of the same receptor. This property allows, for example, efficient targeting of the B-cell lymphomas, even in the presence of relatively high level of circulating idiotype antibody.
- Based on the major feature of high avidity binding, especially for the surface immobilized antigens, the inventive oligomers can also be used as an inhibitor of protein-protein interactions, especially those occurring on the cell surface and in solution.
- Heteropentamers can be produced by reassociating two or more different units of the inventive oligomers together. These heterodimers can bind to different epitopes on the same or different molecules and, e.g., can be used as a chelating and/or crosslinking agent.
- The C-terminus of the molecules can be modified easily, e.g. by introducing peptide ligands with second specificity, a cytotoxic tail, a marker to identify acceptors, or a His-tail to chelate different toxic drugs (e.g. heavy metals) in order to deliver them to the target cells where they can be internalized, via an internalized surface receptor (e.g. immunoglobulin receptor) and kill the target cells.
- It is possible, for example, to isolated a panel of C1q specific peptide ligands which can be used to produce a bispecific form of the inventive oligomers capable of C1 q fixation and complement activation or complement inhibition.
- Fusion of the peptide ligands to an Fc receptor results in a Nt-Ct bispecific form of the inventive oligomers capable of imitating the function of an Fc region from immunoglobulin.
- Frameworks of the inventive oligomers can also be used to create random peptide libraries displayed on the filamentous bacteriophages. This allows rapid selection of the phage displayed inventive oligomers which then can be produced in a soluble form, in a suitable host.
- Due to the relatively short length of the monomer polypeptide chain, the inventive oligomers can be synthesized chemically (e.g. standard Fmoc peptide chemistry, synthesis starts from the C-terminus). The N-terminal position of the peptide ligands allows to synthesized first the core molecule (e.g. the pentamerization domain and a linker) and then split the sample and continue synthesis with different peptide sequences on the N-terminus.
- The same principle of chemical synthesis can be used to produce synthetic peptide library displayed on the inventive oligomers. Namely, synthesizing the N-terminal peptides at random (e.g. by Fmoc chemistry as described above), one can obtain a library of the peptides displayed on the inventive oligomer framework. This library can be absorbed on the target molecules or cells, eluted, purified (e.g. using 6xHis tag) and the N-terminal peptide can be sequenced.
- Furthermore, a framework of the inventive oligomers can be used to oligomerize peptides representing known B-cell epitope in order to obtain an efficient vaccines. Addition of different peptides known to enhance the immune response (such as T-helper epitopes or CR binding peptides derived from C3d) is also possible.
- The inventive units can be expressed directly in a genetically engineered multicellular organism, in order to provide an *in vivo* production of the inventive oligomers.
- The inventive units can be expressed *in vitro* using established transcription-translation systems.
- The inventive units may be used *in vitro* as one of the binding reagents in enzyme immunoassays.
- The inventive units can be used to induce apoptosis.
- Due to the solubility and the high specificity of the compounds they can be used in gene therapy, e.g., to target certain receptors for activation or deactivation; or, if connected to toxic compounds, for their destruction.

- Genes encoding the inventive units may be intracellularly expressed in order to inhibit the binding of transcription factors or gene regulating molecules as well as inhibit enzymatic activities.
- The inventive units may be used to inhibit enzymatic activity or adhesion in order to prevent tumor metastization.
- Furthermore, the epitopes of the inventive units can be used for vaccine development.
- Due their high tendency for oligomerization the Cartilage Oligomeric Matrix Protein can be used generally for the pentamerization of low molecular weight compounds or peptides that are not part of the Cartilage Oligomeric Matrix Protein.

Examples

**Peptide synthesis**. Peptides are synthesized using standard Fmoc solid phase chemistry on an Applied Biosystems 431A peptide synthesizer, lyophilized, re-dissolved in 50% acetic acid, purified by gel filtration through a G-25 sephadex column and analyzed by mass spectroscopy. For competition studies peptides are dissolved in PBS, adjusted to neutral pH, and peptide concentrations are determined by the method of Waddell based on an absorption difference at 215 and 225 nm.

**Bacterial strains**. *E. coli* TG1 (Wertman et al., (1986)- Gene 49, 253-262) is used for propagation of plasmids and phages and *E. coli* SG13009 (QIAEX) is used for production of fusion proteins.

**Labeling**. Typically, Pab-S (17 μg, 0.2 nmol), B1 IgG (75 μg, 0.5 nmol) or B1 Fab' (50 μg. 1 nmol) are labeled in PBS with 100 μCi (1 μCi = 37 MBq) of $^{125}$I in a in Iodo-Gen (Biorad, 10 μg) coated tubes for 20 min (2 h for Pab-S) at 4°C. Uncoupled iodine is removed by gel filtration on a PD-10 column (Pharmacia). About 40% of the radioactivity for Pab-S and 70% for B1 IgG and B1 Fab' is recovered. Specific activity ranges from 70 to 200 μCi/nmol.

**RIA competition on plastic.** Polyvinil 96-well plates are coated with BCL1 IgM at 3 μg / ml in PBS for 16 h at 4°C and blocked with 2% MPBS for 1 h at 37°C. Different amounts of peptides are incubated with 10 nCi of $^{125}$I labeled B1 IgG for 1 h at 37°C. The wells are washed with PBS containing 0.1 % of Tween-20 and the amounts of radioactivity retained is measured with an automated multichannel Cobra II γ-counter.

Example 1: Selection of peptides specific for BCL1 surface immunoglobulin receptor.

**Cells and antibodies**. The BALB/c derived B cell lymphoma $BCL_1$ (Efimov et al., (1994) FEBS Letters 341, 54-58), and the mouse hybridoma B1, secreting an anti-idiotype monoclonal $IgG_1$ antibody (B1 IgG), are kindly provided by Dr. Kris Thielemans (Medical School, VUB, Brussels, Belgium). $BCL_1$ cells are propagated in BALB/c mice using i.p. injection of $10^6$ cells. The $BCL_1$ soluble IgM is purified from the serum of a splenomegalic mouse by precipitation with 50% ammonium sulfate, followed by anion exchange and gel filtration chromatography (Mono Q and Superdex 200, Pharmacia). The B1 IgG is purified by protein G-Sepharose (Pharmacia). Fab' fragments are obtained by limited digestion with pepsin, followed by reduction and alkylation, using standard methods.

**Peptide selection.** Peptide ligands specific for mouse B-cell lymphoma ($BCL_1$) idiotype, selected from three different peptide libraries displayed on two filamentous bacteriophages libraries of about $10^7$ independent members displaying random hexapeptides called Smith (Scott & Smith (1990) Science, 249, 386-390), and Doorbar (Doorbar & Winter (1994) J. Mol. Biol., 244, 361-369), are used. In addition a combinatorial library of about $10^{12}$ independent members displaying a tandem of random decapeptides, called Fisch (Fisch et al., Proc. Natl. Acad. Sci. USA (1996), 93, 7761-7766), is used.

Screening of the phage display libraries is performed essentially as described by Fisch et al., (Proc. Natl. Acad. Sci. USA (1996), 93, 7761-7766). Briefly, the purified $BCL_1$ IgM is coated on immunotubes (Nunc) at 50 μg/ml in PBS (50 mM phosphate, 150m M NaCl, pH 7.4). About $10^{12}$ colony forming units (c.f.u.) of phage from each library are used for each round of selection. After three rounds of panning, individual clones are isolated, and binding of phage to $BCL_1$ IgM coated on plastic (3μg/ml) is measured by ELISA (Barrett et al., (1992) Anal. Biochem. 204, 357-364) using horseradish peroxidase conjugated anti-M13 antibody (Pharmacia). Plates coated with mouse IgG or with human IgM, are used as negative controls. Specific inhibition of phage binding to $BCL_1$ IgM, is performed by addition of B1 IgG at 100 μg/ml. The DNA fragments encoding the selected peptides are amplified by PCR and sequenced as described Fisch et al., (Proc. Natl. Acad. Sci. USA (1996), 93, 7761-7766).

Specific phages are selected on the purified $BCL_1$ IgM, individual phage clones are isolated, and the DNA fragments encoding peptides are amplified by PCR and sequenced (Table 1). Interestingly, two distal Cys residues, potentially capable of forming a disulfide bonded loop, are found in the hexapeptide selected from the Smith library. Idiotype specificity of selected peptides is demonstrated by inhibition of phage binding with the anti-idiotype antibody, B1 IgG.

Table 1 Amino acid sequences of selected idiotype specific peptide ligands for BCL1 immunoglobulin receptor.

| Library / sequence* | Selected peptides† | Sequ ID | name |
|---|---|---|---|
| Doorbar / X6 - PNDKYE - g3p | ***SVWRWL****PYDKYE*‡ | 1 | D |
| Smith / ADGA - X6 - g3p | *ADGA****CRNPWC*** | 2 | S |
| Fisch / X10- ALLRY-X10 - g3p | ***TAAGLCEFDQ***ALLRY**TCPT**¶ | 3 | F |

* Names and amino acid sequences of the phage displayed peptide libraries; X6 and X10 denotes random hexa- and deca-peptide correspondingly.
† Peptides selected from random libraries are in bold type, framework residues are in normal type, corresponding synthetic peptides are in italic type.
‡ An unusual N to Y substitution in the constant part of the peptide from Doorbar library is observed.
¶ First ten amino acid of the peptide selected from Fisch library was synthesized.

Synthetic peptides corresponding to the selected sequences are prepared as outlined in Table 1. Cysteins containing peptide S is completely oxidized on air loosing two hydrogen atoms, as shown by Mass Spectroscopy, which is consistent with the cyclization of the peptide. All synthetic peptides are tested for binding inhibition of $^{125}$I labeled B1 IgG to $BCL_1$ IgM coated on plastic. The D and the S peptides inhibit binding with an $IC_{50}$ of about 60 $\mu$M and 200 $\mu$M, respectively. No inhibition is seen with peptide F up to 2mM, which is used, thereafter, as a negative control. Interestingly, substitution of Cys residues by Ser in peptide S results in a 10 fold loss of affinity, suggesting that disulfide bounded turn conformation of the peptide is favorable for binding to $BCL_1$ IgM idiotype.

Example 2: Pentabody. Molecular design and gene construct

Pentabody fusion protein, is designed consisting of four distinct parts.

- First, a selected peptide specific for $BCL_1$ IgM idiotype represented the N-terminal binding domain.
- Second, a 24 amino acid sequence selected from a long camel Ig hinge region (Hamers-Casterman et al., (1993) Nature 363, 446-448) is placed to provide a space necessary for multivalent binding.
- Third, the hinge region is followed by a 55 amino acid long pentamerization domain, a modification of the described coil-coiled COMP assembly domain (Slavin & Strober (1978) Nature 272, 624-626), known to spontaneously form a five-stranded $\alpha$-helical bundle. Two cystein residues present at the C-terminal of the wild type sequence allowed the formation of inter-chain disulfide bridges. Based on the recently described model of COMP assembly domain (Kajava (1996) Proteins 24, 218-226) two substitutions Lys29Cys and Ala30Cys are introduce in the modified version, making a possible the formation of additional disulfide bonds near the N-terminal part of the assembly domain in order to further stabilize the $\alpha$-helical bundle.
- Forth, a sequence encoding six histidine residues is placed at the C-terminus of the fusion molecule to facilitate the protein purification *via* the metal-chelating affinity chromatography.

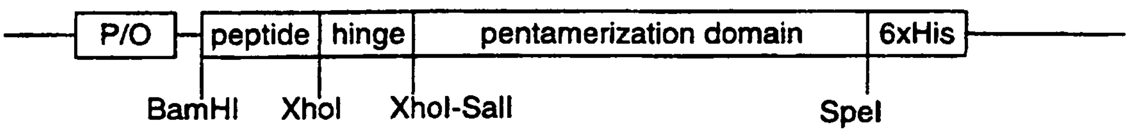


The chimeric genes coding for the different Pab are constructed as designed (see below) and named Pab-S, Pab-D and Pab-F, where the last letters stay for the S, D, and F peptide, respectively (see table 1).

Essentially, the DNA sequences encoding the $BCL_1$ idiotype specific peptides, as well as the hinge region, are assembled from oligonucleotide duplexes. The COMP pentamerization domain is amplified by PCR simultaneously introducing point mutations, and the fusion genes are assembled into a modified pDS-78 expression vector.

**Plasmid construction.** The construction of plasmid p3bCOMP encoding a 64 amino acid COMP assembly domain is described in (Slavin & Strober (1978) Nature 272, 624-626). All further constructs are made using standard methods of DNA manipulation (Sambrook et al., (1990) Molecular Cloning: A Laboratory Manual (Cold Spring Harbor Lab. Press, Painview, NY)). The DNA fragment encoding the COMP domain (residues 26-80, amino acids numbering as in Slavin & Strober (1978) Nature 272, 624-626) is amplified by PCR from p3bCOMP template using COMP-Xho-BACK (5'-AG ATC CTC GAG GGT GGA GAC TGC TGC CCA CAG ATG CTT AGA; SEQ ID NO 4) and COMP-Spe-FOR (5'GC ACT AGT AGA ACC GCC ACC CGG GGT; SEQ ID NO 5) primers. Thus the resulting product contains two amino acid substitutions Lys29Cys and Ala30Cys as well as XhoI and SpeI sites at the 5' and 3' end, respectively. The DNA duplexes encoding the peptides S, F, and D, are prepared by annealing the oligonucleotides S-up (5'GA TCC GCT GAC GGC GCT TGC CGT ACC CCG TGG TGC GGT C; SEQ ID NO 6) with S-low (5'TC GAG ACC GCA CCA CGG GTT ACG GCA AGC GCC GTC AGC G; SEQ ID NO 7), F-up (5'GA TCC ACT GCT GCA GGT CTG TGC GAA TCC GAC CAG C; SEQ ID NO 8) with F-low (5'TC GAG CTG GTC GAA TTC GCA CAG ACC TGC AGC AGT G; SEQ ID NO 9) and D-up (5'GA TCC TCT GTT TGG CGT TGG CTG CCG TAC GAC AAA TAC GAA C; SEQ ID NO 10) with D-low (5'TC GAG TTC GTA TTT GTC GTA CGG CAG CCA ACG CCA AAC AGA G; SEQ ID NO 11). All three duplexes contain BamHI and XhoI cohesive ends on 5' and 3' end, respectively. By means of three-part ligation, the duplexes encoding peptides and the PCR amplified COMP domain, restricted with XhoI and SpeI enzymes, are joint together in the pDS78 vector, linearized with BamHI and SpeI enzymes, in front of a 6 histidine tail present in the vector (Stuber et al., in Immunological Methods, eds. Lefkovits, I. & Perris, B. (Academic Press. New York), pp.121-152). This generated pSC6H pDC6H and pFC6H plasmids which encode S, D and F peptides respectively. A DNA fragment encoding the 24 amino acid hinge region [$(PQ)_2PK(PQ)_4PKPQPK(PE)_2$], called PX, is prepared by annealing of PX-up (5TC GAG CGG CAG CCG CAG CCG AAA CCG CAG CCG CAG CCG CAG CCG CAG CCG AAA CCG CAG CCG AAA CCG GAA CCG GAA G; SEQ ID NO 12) and PX-low (5TC GAC TTC CGG TTC CGG TTT CGG CTG CGG TTT CGG CTG CGG CTG CGG CTG CGG CTG CGG TTT CGG CTG CGG CTG CGG C; SEQ ID NO 13) oligonucleotides encoding the plus and minus strands of the duplex and contain XhoI and SalI cohesive ends on 5' and 3' end, respectively. The PX duplex is ligated into XhoI linearized, dephosphorylated pSC6H pDC6H and pFC6H plasmids, to generate the expression vectors pSPXC6H (DSM 11236), pDPXC6H and pFPXC6H, respectively. The final constructs are verified by dideoxynudeotide sequencing using Sequenase 2.0 (United States Biochemical).

Example 3: Expression and purification of gentabodies.

Pab fusion proteins are expressed in *E. coli* SG13009. The cultures are grown on a shaker at 37°C up to $OD_{600}$ ~0.5, then 1 mM IPTG is added to induce protein synthesis, followed by further 4 h incubation at 30°C. Bacteria are collected by centrifugation (8000 x g, 15 min, 4°C) and frozen at -70°C. Bacterial pellet is resuspended in PBS pH 7.4, 1mM EDTA, 1 mg/ml lysozyme, incubated for 30min at room temperature and subjected to three rounds of freezing/thawing (liquid nitrogen/37°C). The lysate is incubated for 15 min at RT with 0.1 mg/ml of DNAse I and after centrifugation (23,000 x g, 15 min, 4°C) the supernatant is collected. Imidazol is added to a final concentration of 5 mM and the recombinant protein is absorbed on 2ml of Ni-NTA resin (QIAGEN), equilibrated in 5 mM imidazol, PBS pH 7.4. After extensive wash with PBS containing 5 mM and 20 mM imidazol, retained proteins are eluted with PBS containing 250 mM imidazol. After extensive dialysis against PBS, 1 mM EDTA, proteins are concentrated 5 times with Centriprep 10 concentrator (Amicon), aliquoted and stored at -20 °C.

The Pab-S and Pab-F fusion proteins are expressed at high levels (> 30 mg/l), allowing efficient one step metal-chelating affinity chromatography under native conditions. The amount of soluble Pab-D protein is lower than that of Pab-S and Pab-F.

Affinity purified Pab-S and Pab-F molecules are fractionated by FPLC gel filtration. A single elution peak corresponding to a protein of about 85 kDa is observed for Pab-S, whereas two major elution peaks corresponding to the proteins of about 90 and 180 kDa are observed for Pab-F. The high molecular weight peak presumably resulted from dimer formation via unsaturated cystein present in peptide F. In both cases, fractions of the elution peak corresponding to a 85-90 kDa protein are collected, pooled and used for binding studies.

Example 4: Equilibrium binding studies.

Cell dilution experiments and homogenous competition curves (i.e. competition for the cell binding between the labeled and the unlabeled forms of the same ligand) are described by a monovalent equilibrium model. The free (F) and bound (B) radioactivity is corrected from the non-immunoreactive fraction (NIF) and non-specific binding (NSB) respectively. Both are expressed in mol/l, taking into account the specific activity of each isotopic dilution (B and F, respectively). The parameters Kd, R, NSB and NIF, where R is the molarity of binding sites and Kd is the dissociation equilibrium constant, are fitted by non-linear regression of the corrected Scatchard equation

$$\frac{B-NSB}{F-NIF}=\frac{R}{Kd}-\frac{(B-NSB)}{Kd}$$

to the experimental data.

To determine maximal immunoreactivity, $^{125}$I-labeled Pab-S, B1 IgG or B1 Fab' (20 nCi) are incubated with serial dilutions of freshly harvested BCL1 cells (0.3 to 100 x $10^5$ cells per 100 μl). For competition assays, the labeled compounds are incubated with serial dilution of unlabeled competitors and BCL1 cells (25, 50 or 100 x $10^5$ per 100 μl). Comparative experiments are performed the same day with the same batch of cells, in 96-wells plates, in triplicates, in a final volume of 150 μl of PBS supplemented with 1mg/ml of Bovine Serum Albumin (BSA), at 4°C, under agitation and for 2.5 h. After centrifugation, the amounts of both free and bound $^{125}$I are measured as above from an aliquot of the supernatant and of the pellet washed once with PBS (4°C).

In preliminary solid phase competition assay Pab-S is found to compete the binding of $^{125}$I labeled B1 IgG to $BCL_1$ IgM idiotype coated on plastic. However, since spatial arrangement of target molecule on the surface may influence multivalent binding, the equilibrium binding parameters of Pab-S are determined directly on live $BCL_1$ cells, which represent a more relevant biological surface. Purified Pab-S is labeled with $^{125}$I and is shown to bind to BCL1 idiotype on the cell surface. It can be competed by unlabeled Pab-S, B1 IgG, and by a much higher concentration of peptide S, but not by the control Pab-F pentamer. The competitions of $^{125}$I- labeled Pab-S binding by unlabeled Pab-S at 3 different cell concentration and a cell dilution experiment are used together for curve fitting to obtain the equilibrium binding parameters.

The apparent equilibrium binding constant of Pab-S is found to be similar to that of B1 IgG (around 1nM) which represents a 2x$10^5$ fold increase in avidity compared to the peptide S itself ($IC_{50}$ around 200 μM). As control, the same binding experiments are performed with $^{125}$I labeled $B_1$ IgG and B1 Fab' fragment. The obtained constants are consistent with already published data. The Scatchard equilibrium binding constants are:

| Fragment | Kd [nM] |
|---|---|
| Pab-S | 1.0 ± 0.4 |
| IgG | 0.8 ± 0.4 |
| I Fab' | 3.5 ± 0.1 |

The lower molarity of binding sites found for Pab-S compared to B1 IgG and Fab provides evidence for a multivalent nature of Pab-S binding.

In another series of experiments we compare the capacity of soluble $BCL_1$ IgM to competed the binding of either Pab-S or B1 IgG to $BCL_1$ cells. The results showed that much higher concentration of soluble $BCL_1$ IgM is needed to compete for Pab-S as compared to B1 IgG (100 *vs* 4 nM for 80% displacement). These data indicate a higher avidity of Pab-S for the cell surface-immobilized $BCL_1$ IgM, compared to the soluble $BCL_1$ IgM. Importantly, no inhibition of Pab-S binding to $BCL_1$ IgM idiotype is observed in the presence of up to 30 % (v/v) of human or mouse BALB/c serum.

Example 5: Biochemical characterization of Pab-S chimeric protein.

The concentration of purified Pab-S is determined by the method of Waddell, based on an absorption difference at 215 and 225 nm as well as by Bradford protein assay (Bio-Rad). Pab-S molecule is characterized using FPLC gel filtration, SDS-PAGE and CD spectrometry. Oxidized form of Pab-S with inter-chain disulfide bonds is formed by air oxidation during the purification procedure and dialysis against PBS. Completely reduced form is obtained by incubation with 100 mM DTT at 37°C for 30 min, followed by extensive dialysis against PBS, 1 mM EDTA, 1mM β-mercaptoethanol.

Under non-denaturing conditions, a single elution peak, corresponding to a protein of about 85 kDa is detected for both oxidized and reduced forms of Pab-S after gel filtration on Superdex G200 column (equilibrated in PBS, 1 mM EDTA, ± 1mM β-mercaptoethanol, elution is monitored at 280 nm with an integrated UV detector (the proteins are analyzed on 10-15% gradient). Under denaturing conditions (Pab-S is denatured by boiling for 20 min in 4 M urea and 100 mM DTT and renatured by extensive dialysis against PBS, 1 mM EDTA, 1 mM β-mercaptoethanol) in SDS-PAGE, a major protein with apparent molecular weight of about 85 kDa is observed for the oxidized form of Pab-S molecule, whereas a protein with apparent molecular weight of about 17 kDa is observed for the reduced form of Pab-S, in agreement with a covalent pentameric structure. A minor band with apparent mobility of about 68 kDa observed for the oxidized Pab-S corresponds to a partially reduced pentamer, where only four chain out of five are covalently link by disulfide bridges. From the analysis of the CD spectrum of Pab-S pentamer an α-helical content of about 54 % is determined by fitting the experimental data with a set of reference proteins as described in Vogel (Biochemistry (1987), 26, 4562-72). This is in a good agreement with the length of the α-helical coiled-coil domain within the entire molecule. Taken together these data indicate that Pab-S molecule is a stable homopentamer of about 85 kDa with a monomer subunits of about 17 kDa held together by in α-helical coiled-coil bundle, where the five chains are covalently crosslinked by disulfide bonds.

It is known that coiled coil structures can undergo reversible denaturation. Indeed, the Pab-S molecule is denatured in urea at 95°C under reducing conditions and refolded into the pentamer by simple dialysis against PBS, as demonstrated by FPLC gel filtration of renatured Pab-S labeled with $^{125}$I. Noteworthy, renaturation of Pab-S restores full binding activity as shown by binding competition on BCL1 cells.

To visualize the spatial arrangement of Pab-S molecular modeling of its three-dimensional structure is undertaken. Pab-S molecule is modeled using the computer graphics program TOURBO-FRODO (Roussel & Cambillan (1989) in Silicon Graphics Geometry Partner Directory (Fall 1989), eds. Silicon Graphics (Silicon Graphics, Mountain View, CA), pp.77-78). The structure is further refined by the X-PLOR program version 3.1 (Brunger. (1992) in X-PLOR version 3.1 A system for X-ray Crystallography and NMR, (New Haven, Yale University Press)) using the following procedure: a 5 ps molecular dynamics simulation at 300 K and then 1,000 steps of conjugate gradient minimization, carried out with 1/r dependent dielectric constant.

The stereochemical analysis shows that frequently occurring type I conformation of β-turn allows formation of disulfide bond between the two cysteins of peptide S. This conformation is chosen, as the most likely, for the peptide ligand. For the hinge region a molecular dynamic simulation with subsequent energy minimization resulted in a set of possible conformations. Out of it, a "good-looking" conformation is arbitrary chosen. Finally, previously modeled five-stranded α-helical coiled-coil structure is taken for the pentamerization domain. The analysis suggests a fivefold symmetrical structure of Pab-S protein. The analysis of the modeled Pentabody structure shows that such a molecule should be capable of simultaneous binding to five surface receptor, provided they are located up to 80 Å apart from each other. This criterion is largely satisfied in the case of the surface immunoglobulin receptor, whose variable domains can be spaced at about 50 Å, judging by their van der Waals contact radii.

Example 6:Use of pentabody in *in vitro* for assays

To measure the level of an antigen of interest such as prostrate specific antigen (PSA) which is an enzyme of the kallikrein family, peptides displayed on a phage display library may be screened for those binding specifically the antigen of interest, such as PSA, with a great specificity and avidity. Screening may be as described in the examples above, particularly example 1. Pentabody expressing the selected peptide(s) in pentameric form may be constructed, expressed and purified as described in the examples above, particularly examples 2 and 3. The selected pentabodies may be labeled with a marker enzyme such as peroxidase.

Antibodies specific for the antigen of interest, such as PSA, may be immobilized on a solid phase such as plates or polystyrene balls. The immobilized, unsolubized antibodies may be incubated with the test fluid, such as serum or other biological fluid for the first step of immunoabsorption. After incubation, the solid phase may be washed.

The selected pentabody labeled with a marker enzyme may be incubated with the immunoabsorbed antigen, such as PSA. Then the substrate for the marker enzyme conjugated to the pentabody and a chromogen may be added. The optical density may then be measured. The optical density of the chromogen will be proportional to the amount of antigen, such as PSA, present. The assays of the invention may also comprise radioimmunassays using the pentabodies of the invention, the above approach and methods known in the art.

Deposition of Microorganisms

The following microorganisms are deposited according to the Budapest Treaty with the DSM - Deutsche Sammlung von Mikroorganismen und Zellkulturen GmbH, Mascheroder Weg 1b, D-38124 Braunschweig:

*E. coli* SG13009 pSXC6H 21.10.1996 DSM11236

## Claims

**1.** . An oligomer comprising 2 or more units, wherein each unit comprises

(i) an oligomerizing domain of the Cartilage Oligomeric Matrix Protein capable of assembling monomeric units; and
(ii) a protein domain or a low molecular weight compound,

wherein said protein domain or a low molecular weight compound are not of the Cartilage Oligomeric Matrix Protein.

**2.** . An oligomer according to Claim 1, wherein said protein domain is a binding domain capable of binding to a target molecule.

**3.** . An oligomer according to Claim 2, wherein the oligomerising domain and the binding domain are connected via a spacer.

4. . An oligomer according to claim 2 or 3 consisting of 5 units.

5. . An oligomer according to any one of claims 2-4, wherein the target molecule is an antibody or a receptor.

6. . An oligomer according to any one of claims 2-5, wherein each of said units has less than 600 amino acids.

7. . An oligomer according to any one of claims 2-6, wherein at the C-terminus of some or all units a further functional domain is attached.

8. . An oligomer according to any one of claims 2-7, wherein the individual units oligomerize spontaneously.

9. . An oligomer according to any one of claims 2-8, wherein the oligomerizing domain is the assembly domain of Cartilage Oligomeric Matrix Protein (COMP) comprising of 64 amino acids from residues 26-80 or a fragment thereof retaining the capacity to oligomerize.

10. . An oligomer according to claim 8 wherein the oligomerising domain is a mutant of the Cartilage Oligomeric Matrix Protein (COMP) assembly domain introducing two substitutions of Lys-29 and Ala-30 which are both changed to Cys.

11. . An oligomer according to any one of claims 2-10, wherein said oligomer is a homo-oligomer in that said units have the same specificity for binding to a target molecule.

12. . An oligomer according to any one of claims 2-10, wherein said oligomer is a hetero-oligomer in that said units have a different specificity for binding to a target molecule.

13. . An oligomer according to any one of claims 1-12 wherein the binding domain is selected from SEQ.ID. No.1. SEQ ID No.2, or SEQ. ID No.3.

14. . An oligomer according to any one of claims 1-13 wherein said spacer is selected from SEQ.ID.No. 12 or SEQ ID No. 13.

15. . Use of an oligomer according to any one of claims 1-14 in vitro for identification and/or marking of target molecules.

16. . Use of an oligomer according to any one of claims 1-14 in vitro for eukaryotic cell, bacteria or viruses targeting.

17. . Use of an oligomer according to any one of claims 1-14 in vitro for cell targeting.

18. . Use according to claim 17 for in vitro targeting of B-cell lymphomas.

19. . Use of an oligomer according to any one of claims 1-14 for the in vitro inhibition of protein-protein interactions.

20. . Use of an oligomer according to any one of claims 1-14 in vitro as a chelating agent.

21. . Use of an oligomer according to any one of claims 1-14 in vitro as a crosslinking agent.

22. . Use of oligomers according to any one of claims 1-14 for the construction of libraries.

23. . Use of oligomers according to any one of claims 1-14 in vitro for the induction of apoptosis.

24. . Use of oligomers according to any one of claims 1-14 in vitro for the intracellular inhibition of transcription factor binding, gene regulating molecules and/or enzymatic activities.

25. . Use of oligomers according to any one of claims 1-14 in vitro for prevention of tumor metastatization.

26. . Use of oligomers according to any one of claims 1-14 in vitro as one of the binding reagents in an enzyme immunoassay.

27. . Use of oligomers according to any one of claims 1-14in vitro as one of the binding reagents in radioimmunoassays.

**28.** . A method for the production of an oligomer according to any one of claims 1-14, comprising the use of a monomer unit comprising

(i) an oligomerizing domain of the Cartilage Oligomeric Matrix Protein capable of assembling monomeric units; and
(ii) a protein domain or a low molecular weight compound,

wherein said protein domain or a low molecular weight compound are not of the Cartilage Oligomeric Matrix Protein.

**29.** . An oligomer according to any one of claims 2-14, wherein the epitopes are used for vaccine development.

**Patentansprüche**

**1.** Oligomer, das zwei oder mehr Einheiten umfasst, worin jede Einheit umfasst

(i) eine Oligomerisationsdomain des oligomeren Knorpel-Matrix-Proteins (Cartilage Oligomeric Matrix Protein - COMP), das zu einer Sammlung von Monomereinheiten befähigt ist, und
(ii) eine Proteindomain oder eine niedermolekulare Verbindung,

worin die Proteindomain oder eine niedermolekulare Verbindung nicht vom oligomeren Knorpel-Matrix-Protein stammen.

**2.** Oligomer nach Anspruch 1, worin die Proteindomain eine Bindungsdomain ist, die zur Bindung an ein Zielmolekül befähigt ist.

**3.** Oligomer nach Anspruch 2, worin die Oligomerisationsdomain und die Bindungsdomain über einen Spacer verbunden sind.

**4.** Oligomer nach Anspruch 2 oder 3, das aus 5 Einheiten besteht.

**5.** Oligomer nach einem der Ansprüche 2 bis 4, worin das Zielmolekül ein Antikörper oder ein Rezeptor ist.

**6.** Oligomer nach einem der Ansprüche 2 bis 5, worin jede dieser Einheiten weniger als 600 Aminosäuren aufweist.

**7.** Oligomer nach einem der Ansprüche 2 bis 6, worin an den C-Terminus einiger oder aller Einheiten eine weitere funktionale Domain gebunden ist.

**8.** Oligomer nach einem der Ansprüche 2 bis 7, worin die einzelnen Einheiten spontan oligomerisieren.

**9.** Oligomer nach einem der Ansprüche 2 bis 8, worin die Oligomerisationsdomain die Sammlungsdomain des oligomeren Knorpel-Matrix-Proteins (COMP) ist, die 64 Aminosäuren der Reste 26 bis 80 oder ein Fragment hiervon umfasst, wodurch die Fähigkeit zur Oligomerisation beibehalten wird.

**10.** Oligomer nach Anspruch 8, worin die Oligomerisationsdomain eine Mutante der Sammlungsdomain des oligomeren Knorpel-Matrix-Proteins (COMP) ist, die zwei Substitutionen von Lys-29 und Ala-30 einführt, wovon beide zu Cys verändert werden.

**11.** Oligomer nach einem der Ansprüche 2 bis 10, worin das Oligomer ein Homooligomer ist, bei dem die Einheiten eine gleiche Spezifität für eine Bindung an ein Zielmolekül haben.

**12.** Oligomer nach einem der Ansprüche 2 bis 10, worin das Oligomer ein Heterooligomer ist, bei dem die Einheiten eine unterschiedliche Spezifität für eine Bindung an ein Zielmolekül haben.

**13.** Oligomer nach einem der Ansprüche 1 bis 12, worin die Bindungsdomain ausgewählt ist aus SEQ.ID Nr. 1, SEQ ID Nr. 2 oder SEQ ID Nr. 3.

**14.** Oligomer nach einem der Ansprüche 1 bis 13, worin der Spacer ausgewählt ist aus SEO.ID Nr. 12 oder SEQ ID Nr. 13.

**15.** Verwendung eines Oligomers nach einem der Ansprüche 1 bis 14 in vitro für eine Identifikation und/oder Markierung von Zielmolekülen.

**16.** Verwendung eines Oligomers nach einem der Ansprüche 1 bis 14 in vitro für eukaryotische Zellen, Bakterien oder Viren als Ziele.

**17.** Verwendung eines Oligomers nach einem der Ansprüche 1 bis 14 in vitro für eine Zelle als Ziel.

**18.** Verwendung nach Anspruch 17 in vitro für B-Zellenlymphome als Ziel.

**19.** Verwendung eines Oligomers nach einem der Ansprüche 1 bis 14 für die in vitro Inhibition von Protein-Protein-Interaktionen.

**20.** Verwendung eines Oligomers nach einem der Ansprüche 1 bis 14 in vitro als ein Chelatbildner.

**21.** Verwendung eines Oligomers nach einem der Ansprüche 1 bis 14 in vitro als ein Vernetzungsmittel.

**22.** Verwendung von Oligomeren nach einem der Ansprüche 1 bis 14 für die Konstruktion von Bibliotheken.

**23.** Verwendung von Oligomeren nach einem der Ansprüche 1 bis 14 in vitro für die Induktion einer Apoptose.

**24.** Verwendung von Oligomeren nach einem der Ansprüche 1 bis 14 in vitro für die intrazellulare Inhibition einer Transkriptionsfaktorbindung, von Genregulationsmolekülen und/oder von enzymatischen Aktivitäten.

**25.** Verwendung von Oligomeren nach einem der Ansprüche 1 bis 14 in vitro für die Prävention einer Tumormetastatisierung.

**26.** Verwendung von Oligomeren nach einem der Ansprüche 1 bis 14 in vitro als eines der Bindungsreagenzien in Enzymimmunassays.

**27.** Verwendung von Oligomeren nach einem der Ansprüche 1 bis 14 in vitro als eines der Bindereagenzien in Radioimmunassays.

**28.** Verfahren zur Herstellung eines Oligomers nach einem der Ansprüche 1 bis 14, umfassend die Verwendung einer Monomereinheit, die umfasst

(i) eine Oligomerisationsdomain des oligomeren Knorpel-Matrix-Proteins (Cartilage Oligomeric Matrix Protein - COMP), das zu einer Sammlung von Monomereinheiten befähigt ist, und
(ii) eine Proteindomain oder eine niedermolekulare Verbindung,

worin die Proteindomain oder eine niedermolekulare Verbindung nicht vom oligomeren Knorpel-Matrix-Protein stammen.

**29.** Oligomer nach einem der Ansprüche 2 bis 14, worin die Epitopen für eine Vakzineentwicklung verwendet werden.

## Revendications

**1.** Oligomère comprenant 2 unités ou plus, **caractérisé en ce que** chaque unité comprend :

(i) un domaine oligomérisant de la protéine matricielle oligomérique du cartilage capable d'assembler des unités monomériques ; et
(ii) un domaine protéique ou un composé de faible poids moléculaire,

**caractérisé en ce que** ledit domaine protéique ou le composé de faible poids moléculaire n'appartiennent pas à la protéine matricielle oligomérique du cartilage.

**2.** Oligomère selon la revendication 1, **caractérisé en ce que** ledit domaine protéique est un domaine de liaison capable de se lier à une molécule cible.

3. Oligomère selon la revendication 2, **caractérisé en ce que** le domaine oligomérisant et le domaine de liaison sont reliés via un espaceur.

4. Oligomère selon la revendication 2 ou 3 composé de 5 unités.

5. Oligomère selon l'une quelconque des revendications 2 à 4, **caractérisé en ce que** la molécule cible est un anticorps ou un récepteur.

6. Oligomère selon l'une quelconque des revendications 2 à 5, **caractérisé en ce que** chaque unité possède moins de 600 acides aminés.

7. Oligomère selon l'une quelconque des revendications 2 à 6, **caractérisé en ce qu'**au niveau du terminal C de certaines des unités, ou de toutes les unités, est attaché un autre domaine fonctionnel.

8. Oligomère selon l'une quelconque des revendications 2 à 7, **caractérisé en ce que** les unités individuelles s'oligomérisent spontanément.

9. Oligomère selon l'une quelconque des revendications 2 à 8, **caractérisé en ce que** le domaine oligomérisant est le domaine d'assemblage de la protéine matricielle oligomérique du cartilage (COMP) comprenant 64 acides aminés provenant des résidus 26-80 ou d'un de ses fragments qui conserve la capacité de s'oligomériser.

10. Oligomère selon la revendication 8, **caractérisé en ce que** le domaine oligomérisant est un mutant du domaine d'assemblage de la protéine matricielle oligomérique du cartilage (COMP) introduisant deux substitutions de Lys-29 et Ala-30 qui sont toutes deux changées en Cys.

11. Oligomère selon l'une quelconque des revendications 2 à 10, **caractérisé en ce que** ledit oligomère est un homo-oligomère dans lequel lesdites unités possèdent la même spécificité en vue de se lier à une molécule cible.

12. Oligomère selon l'une quelconque des revendications 2 à 10, **caractérisé en ce que** ledit oligomère est un hétéro-oligomère dans lequel lesdites unités possèdent la même spécificité en vue de se lier à une molécule cible.

13. Oligomère selon l'une quelconque des revendications 1 à 12, **caractérisé en ce que** le domaine de liaison est sélectionné à partir de la SEQ.ID. No.1, SEQ ID No.2, ou SEQ. ID No.3.

14. Oligomère selon l'une quelconque des revendications 1 à 13, **caractérisé en ce que** le espaceur est sélectionné à partir de la SEQ.ID. No.12 ou de la SEQ ID No.13.

15. Utilisation d'un oligomère selon l'une quelconque des revendications 1 à 14 pour l'identification et/ou le marquage *in vitro* des molécules cibles.

16. Utilisation d'un oligomère selon l'une quelconque des revendications 1 à 14 pour le ciblage *in vitro* des cellules eucaryotes, des bactéries ou des virus.

17. Utilisation d'un oligomère selon l'une quelconque des revendications 1 à 14 pour le ciblage *in vitro* des cellules.

18. Utilisation d'un oligomère selon l'une quelconque des revendications 1 à 14 pour le ciblage *in vitro* des lymphomes B.

19. Utilisation d'un oligomère selon l'une quelconque des revendications 1 à 14 pour l'inhibition *in vitro* des interactions protéine-protéine.

20. Utilisation d'un oligomère selon l'une quelconque des revendications 1 à 14 en tant qu'agent chélateur *in vitro.*

21. Utilisation d'un oligomère selon l'une quelconque des revendications 1 à 14 en tant qu'agent de réticulation *in vitro.*

22. Utilisation d'un oligomère selon l'une quelconque des revendications 1 à 14 pour la construction de pharmacothèques.

**23.** Utilisation d'un oligomère selon l'une quelconque des revendications 1 à 14 pour l'induction *in vitro* de l'apoptose.

**24.** Utilisation d'oligomères selon l'une quelconque des revendications 1 à 14 pour l'inhibition intra-cellulaire *in vitro* de la liaison du facteur de transcription, des molécules régulant les gènes et/ou des activités enzymatiques.

**25.** Utilisation d'oligomères selon l'une quelconque des revendications 1 à 14 pour la prévention *in vitro* des métastases tumorales.

**26.** Utilisation d'oligomères selon l'une quelconque des revendications 1 à 14 *in vitro* en tant que réactifs de liaison au cours d'une immuno-analyse enzymatique.

**27.** Utilisation d'oligomères selon l'une quelconque des revendications 1 à 14 *in vitro* en tant que réactifs de liaison au cours d'une radio-immuno-analyse.

**28.** Méthode de production d'un oligomère selon l'une quelconque des revendications 1 à 14 comprenant l'utilisation d'une unité monomère comprenant

(i) un domaine oligomérisant de la protéine matricielle oligomérique du cartilage capable d'assembler des unités monomériques ; et
(ii) un domaine protéique ou un composé de faible poids moléculaire,

**caractérisé en ce que** ledit domaine protéique ou le composé de faible poids moléculaire n'appartiennent pas à la protéine matricielle oligomérique du cartilage.

**29.** Oligomère selon l'une quelconque des revendications 2 à 14, **caractérisé en ce que** les épitopes sont employés pour le développement de vaccins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9531540 A


### Non-patent literature cited in the description


- **EFIMOV et al.** *Federation of European Biochemical Societies Letters,* 1994, vol. 341, 54-58
- **ARGOS.** *Journal of Molecular Biology,* vol. 211, 943-958
- **SLAVIN ; STROBER.** *Nature,* 1978, vol. 272, 624-626
- **WERTMAN et al.** *Gene,* 1986, vol. 49, 253-262
- **EFIMOV et al.** *FEBS Letters,* 1994, vol. 341, 54-58
- **SCOTT ; SMITH.** *Science,* 1990, vol. 249, 386-390
- **DOORBAR ; WINTER.** *J. Mol. Biol.,* 1994, vol. 244, 361-369
- **FISCH et al.** *Proc. Natl. Acad. Sci. USA,* 1996, vol. 93, 7761-7766
- **BARRETT et al.** *Anal. Biochem.,* 1992, vol. 204, 357-364
- **HAMERS-CASTERMAN et al.** *Nature,* 1993, vol. 363, 446-448
- **KAJAVA.** *Proteins,* 1996, vol. 24, 218-226
- **SAMBROOK et al.** Molecular Cloning: A Laboratory Manual. Cold Spring Harbor Lab. Press, 1990
- **STUBER et al.** Immunological Methods. Academic Press, 121-152
- **VOGEL.** *Biochemistry,* 1987, vol. 26, 4562-72
- **ROUSSEL ; CAMBILLAN.** Silicon Graphics Geometry Partner Directory. 1989, 77-78
- **BRUNGER.** A system for X-ray Crystallography and NMR. Yale University Press, 1992

Sequenzlisting

(1) GENERAL INFORMATION:

(i) APPLICANT:

(A) NAME: CIBA-GEIGY AG
(B) STREET: Klybeckstr. 141
(C) CITY: Basel
(E) COUNTRY: Switzerland
(F) POSTAL CODE (ZIP): 4002
(G) TELEPHONE: +41 61 696 11 11
(H) TELEFAX: + 41 61 696 79 76

(I) TELEX: 962 991

(i) APPLICANT:

(A) NAME: University of Lausanne
(B) STREET: Chemin des Boveresses 155
(C) CITY: Epalinges
(E) COUNTRY: Switzerland
(F) POSTAL CODE (ZIP): 1066
(G) TELEPHONE:
(H) TELEFAX:
(I) TELEX:

(i) APPLICANT:

(A) NAME: Swiss Institute for Allergy and Astma Research
(B) STREET: Obere Stasse 22
(C) CITY: Davos
(E) COUNTRY: Switzerland
(F) POSTAL CODE (ZIP): 7270
(G) TELEPHONE:
(H) TELEFAX:
(I) TELEX:

(i) APPLICANT:

(A) NAME: ISREC
(B) STREET: Chemin des Boveresses 155
(C) CITY: Epalinges
(E) COUNTRY: Switzerland
(F) POSTAL CODE (ZIP): 1066
(G) TELEPHONE:
(H) TELEFAX:
(I) TELEX:

(ii) TITLE OF INVENTION: Oligomers
(iii) NUMBER OF SEQUENCES: 13
(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:

(A) LENGTH: 12 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 1:

Ser Val Trp Arg Trp Leu Pro Tyr Asp Lys Tyr Glu
1 5 10

(2) INFORMATION FOR SEQ ID NO: 2:

(i) SEQUENCE CHARACTERISTICS:

(A) LENGTH: 10 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 2:

Ala Asp Gly Ala Cys Arg Asn Pro Trp Cys
1 5 10

(2) INFORMATION FOR SEQ ID NO: 3: -

(i) SEQUENCE CHARACTERISTICS:

(A) LENGTH: 19 amino acids
(B) TYPE: amino acid
(C) STRANDEDNESS: single
(D) TOPOLOGY: linear

(ii) MOLECULE TYPE: peptide
(xi) SEQUENCE DESCRIPTION: SEQ ID NO: 3:

Thr Ala Ala Gly Leu Cys Glu Phe Asp Gln Ala Leu Leu Arg Tyr Thr
1 5 10 15

Cys Pro Thr

(2) INFORMATION FOR SEQ ID NO: 4:

(i) SEQUENCE CHARACTERISTICS:

(A) LENGTH: 41 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 4:
AGATCCTCGA GGGTGGAGAC TGCTGCCCAC AGATGCTTAG A 41

(2) INFORMATION FOR SEQ ID NO: 5:

(i) SEQUENCE CHARACTERISTICS:

(A) LENGTH: 26 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 5:
GCACTAGTAG AACCGCCACC CGGGGT 26

(2) INFORMATION FOR SEQ ID NO: 6:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 6:
GATCCGCTGA CGGCGCTTGC CGTACCCCGT GGTGCGGTC 39

(2) INFORMATION FOR SEQ ID NO: 7:

(i) SEQUENCE CHARACTERISTICS:

(A) LENGTH: 39 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 7:
TCGAGACCGC ACCACGGMT ACGGCAAGCG CCGTCAGCG 39

(2) INFORMATION FOR SEQ ID NO: 8:

(i) SEQUENCE CHARACTERISTICS:

(A) LENGTH: 36 base pairs
(B) TYPE: nucleic acid
(C) STRANDEDNESS : single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 8:
GATCCACTGC TGCAGGTCTG TGCGAATCCG ACCAGC 36

(2) INFORMATION FOR SEQ ID NO: 9:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 9:
TCGAGCTGGT CGAATTCGCA CAGACCTGCA GCAGTG 36

(2) INFORMATION FOR SEQ ID NO: 10:

(i) SEQUENCE CHARACTERISTICS:

(A) LENGTH: 42 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 10:
GATCCTCTGT TTGGCGTTGG CTGCCGTACG ACAAATACGA AC 42

(2) INFORMATION FOR SEQ ID NO: 11:

(i) SEQUENCE CHARACTERISTICS:

(A) LENGTH: 42 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 11:
TCGAGTTCGT ATTTGTCGTA CGGCAGCCAA CGCCAAACAG AG 42

(2) INFORMATION FOR SEQ ID NO: 12:

(i) SEQUENCE CHARACTERISTICS:

(A) LENGTH: 78 base pairs

(B) TYPE: nucleic acid
(C) STRANDEDNESS : single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 12:

TCGAGCGGCA GCCGCAGCCG AAACCGCAGC CGCAGCCGCA GCCGCAGCCG AAACCGCAGC 60

CGAAACCGGA ACCGGAAG 78

(2) INFORMATION FOR SEQ ID NO: 13:

(i) SEQUENCE CHARACTERISTICS:

(A) LENGTH: 78 base pairs
(B) TYPE: nucleic acid
(C) STRANDEDNESS: single
(D) TOPOLOGY: linear

(ii) MOLECULE TYPE: other nucleic acid

(A) DESCRIPTION: /desc = "PCR primer"

(xi) SEQUENCE DESCRIPTION: SEQ ID NO: 13:

TCGACTTCCG GTTCCGGTTT CGGCTGCGGT TTCGGCTGCG GCTGCGGCTG CGGCTGCGGT 60

TTCGGCTGCG GCTGCGGC 78